# EUROPEAN PATENT SPECIFICATION

(11) **EP 3 234 191 B1**
(45) Date of publication and mention of the grant of the patent: **09.09.2020**
(21) Application number: 15823782.6
(22) Date of filing: 11.12.2015
(51) Int. Cl.: C12Q 1/6818

(54) **A METHOD FOR THE COLORIMETRIC DETECTION OF THE AMPLIFICATION OF A TARGET NUCLEIC ACID SEQUENCE**
VERFAHREN ZUM KOLORIMETRISCHEN NACHWEIS VON AMPLIFIZIERUNG EINER ZIELNUKLEINSÄURESEQUENZ
PROCÉDÉ POUR LA DÉTECTION COLORIMÉTRIQUE DE L'AMPLIFICATION D'UNE SÉQUENCE D'ACIDE NUCLÉIQUE CIBLE

(30) Priority: 15.12.2014 IT TO20141037
(43) Date of publication of application: 25.10.2017
(73) Proprietor: Fondazione Istituto Italiano di Tecnologia, 16163 Genova (IT)
(72) Inventor: POMPA, Pier Paolo, I-73100 Lecce (IT); VALENTINI, Paola, I-16131 Genova (IT)
(74) Representative: Comoglio, Elena
(86) International application number: PCT/IB2015/059546
(87) International publication number: WO 2016/097953

(56) References cited:
- WO-A1-01/83823
- JP-A- 2013 059 321
- US-A1- 2002 127 574
- US-A1- 2003 113 737
- US-A1- 2009 155 785
- US-A1- 2014 272 972
- HUA DENG ET AL: "Gold Nanoparticles with Asymmetric Polymerase Chain Reaction for Colorimetric Detection of DNA Sequence", ANALYTICAL CHEMISTRY, vol. 84, no. 3, 7 February 2012 (2012-02-07), pages 1253-1258, XP055208398, ISSN: 0003-2700, DOI: 10.1021/ac201713t cited in the application

### SEQUENCE LISTING

<110> FONDAZIONE ISTITUTO ITALIANO DI TECNOLOGIA
<120> A method for the colorimetric detection of the amplification of a target nucleic acid sequence
<130> PC1421EC-E0100884
<160> 15
<170> PatentIn version 3.5
<210> 1
   <211> 14
   <212> DNA
   <213> artificial
<220>
   <223> universal tag sequence
<400> 1
   taaactctga tgta 14
<210> 2
   <211> 12
   <212> DNA
   <213> artificial
<220>
   <223> universal tag sequence
<400> 2
   aaactctgat gt 12
<210> 3
   <211> 10
   <212> DNA
   <213> artificial
<220>
   <223> universal tag sequence
<400> 3
   aactctgatg 10
<210> 4
   <211> 19
   <212> DNA
   <213> artificial
<220>
   <223> oligonucleotide probe
<400> 4
   tttttatcat catacatca 19
<210> 5
   <211> 19
   <212> DNA
   <213> artificial
<220>
   <223> oligonucleotide probe
<400> 5
   tttttatcat ctgtacctg 19
<210> 6
   <211> 25
   <212> DNA
   <213> artificial
<220>
   <223> oligonucleotide probe
<400> 6
   acatcagagt caacattttt ttttt 25
<210> 7
   <211> 24
   <212> DNA
   <213> artificial
<220>
   <223> oligonucleotide probe
<400> 7
   gagtttacca agtatttttt tttt 24
<210> 8
   <211> 19
   <212> DNA
   <213> artificial
<220>
   <223> oligonucleotide probe
<400> 8
   gtgaattaca agtattttt 19
<210> 9
   <211> 20
   <212> DNA
   <213> artificial
<220>
   <223> oligonucleotide probe
<400> 9
   tgttgactct tttttttttt 20
<210> 10
   <211> 30
   <212> DNA
   <213> artificial
<220>
   <223> forward primer
<400> 10
   acatcagagt ttccagcaca atgaagatca 30
<210> 11
   <211> 19
   <212> DNA
   <213> artificial
<220>
   <223> reverse primer
<400> 11
   gatccacacg gagtacttg 19
<210> 12
   <211> 32
   <212> DNA
   <213> artificial
<220>
   <223> forward primer
<400> 12
   acatcagagt cacttccctc ctcagatcat tg 32
<210> 13
   <211> 19
   <212> DNA
   <213> artificial
<220>
   <223> reverse primer
<400> 13
   gatccacacg gagtacttg 19
<210> 14
   <211> 38
   <212> DNA
   <213> artificial
<220>
   <223> forward primer
<400> 14
   ttggtacctg gtgaattcct tccctcctca gatcattg 38
<210> 15
   <211> 17
   <212> DNA
   <213> artificial
<220>
   <223> reverse primer
<400> 15
   aggaaagaca cccacct 17

## Description

The present invention relates to a method for the detection of the amplification of a target nucleic acid sequence in a liquid sample in which the presence of an amplification product is determined colorimetrically, by exploiting the known property of colloidal gold nanoparticles (AuNPs) to give rise to a visible colour change when, as a result of their aggregation, the interparticle distance becomes less than the size (diameter) of the particles (an effect due to plasmon coupling).

Nucleic acid amplification reactions, and in particular the PCR, are an extremely powerful tool that has revolutionized the molecular biology field in the last thirty years. These techniques allow for the rapid and sensitive identification of a target nucleic acid of interest, even if present in the sample in very low concentrations or diluted in an excess of interfering genetic material. The PCR, and the other known techniques for nucleic acid amplification allow to detect the presence of infectious agents or gene mutations or rearrangements in samples obtained from patients, or to detect the presence of pathogenic contaminants in food or water samples. They also find wide application in the scientific research field and are fundamental for the screening of populations of laboratory animals in order to identify transgenic subjects.

The standard procedure for the verification of the results of a PCR or other nucleic acid amplification technique comprises performing an agarose gel electrophoresis, staining the gel, and finally reading it with a scanner or reader equipped with an excitation lamp at an appropriate wavelength. The working time required for the entire procedure (which entails the preparation of the gel, the electrophoretic run, the staining and washing, the reading, and the interpretation of the results) is estimated at around at least 2-3 hours. The standard agarose gels allow for the analysis of up to 19 samples at a time, therefore, if there is a greater number of samples, the entire procedure must be performed on multiple gels in parallel, which multiplies costs and equipment, or it must be repeated several times, with a considerable increase in working times. Even if these procedures are extremely standardized, they still require expensive reagents, long processing times, and specific instrumentation. These methodologies can also become difficult to carry out in some high throughput applications, such as for example the identification of transgenic animals, in which a reduction in the time of analysis would have a major impact on cost reduction and mitigation of the suffering inflicted on the animals themselves, as well as in the clinical diagnostics field, in which emergency screenings for pathogens causing epidemics may be required.

Cross-linking experiments for colloidal gold nanoparticles (AuNPs) are described in the prior art, in which the AuNPs are functionalized with oligonucleotides and cross-linked by means of complementary oligonucleotides. These experiments exploit the known property of an AuNP solution of undergoing a visible change of colour (from red to blue) when the interparticle distance decreases.

Alivisatos P. A. et al., Organization of "Nanocrystal Molecules" Using DNA, Nature 1996, 6592, 609-611 describes the formation of dimers and trimers of colloidal gold nanoparticles (AuNPs) functionalized with a single-stranded oligonucleotide, through the use of a complementary oligonucleotide.

In Mirkin C. A. et al. A DNA-Based Method for Rationally Assembling Nanoparticles into Macroscopic Materials, Nature 1996, 6592, 607-609, a method is described which provides for the functionalization of two series of AuNPs with non-complementary DNA oligonucleotides bearing thiol groups, which bind to gold. A duplex oligonucleotide with free single-stranded ends ("sticky ends"), which are complementary to the two sequences with which the AuNPs are functionalized, is then added to the AuNP solution, which causes self assembly of the AuNPs into aggregates.

The scheme outlined above requires the functionalization of two series of AuNPs with two different oligonucleotide probes, each complementary to a portion of the target DNA, in such a way that the presence of the target induces AuNP cross-linking and the consequent change of colour of the solution. This scheme is suitable for the detection of short sequences of target DNA, but cannot be used as a universal detector for the amplification of nucleic acids, for the following reasons:
1) for each target, a pair of AuNPs functionalized with oligonucleotide probes specific for the target must be prepared;
2) the change in colour (from red to blue) is greatest with very small interparticle distances, but it gradually becomes less evident as the interparticle distances increase; however, most of the PCR amplification products are longer (typically > 30 base pairs) than the cross-linking oligonucleotide probes which allow a proper plasmon coupling of the AuNPs and a noticeable colour change;
3) the PCR products are double-stranded and must be denatured before hybridization with the complementary oligonucleotide probes immobilized on the AuNPs;
4) even after denaturation, the complementary strand of the target competes with the oligonucleotide probes, which causes a marked reduction in the sensitivity of the assay.

An assay based on the implementation of an asymmetric PCR and hybridization with AuNP probes is described in the scientific literature (Deng H et al. Gold Nanoparticles with Asymmetric Polymerase Chain Reaction for Colorimetric Detection of DNA Sequence, Anal. Chem. 2012, 84, 1253-1258). However, this assay only overcomes the above-mentioned disadvantages in items 3) and 4), thanks to the use of an asymmetric PCR, but it still requires a target-specific functionalization of the AuNPs and only detects moderate colour changes (from red to light purple), probably because of steric hindrance of the relatively long PCR product, which interferes with the aggregation of the AuNPs.

In order to overcome these and other drawbacks of the prior art, the inventors developed methods and related kits as defined in the appended independent claims.

Specific embodiments of the invention are the subject of the dependent claims, the content of which is to be understood as an integral part of the description.

The invention which is the object of the present patent application solves the problems of the prior art by providing a universal detection system for nucleic acid amplification which requires neither specific equipment, nor long execution times, nor the design of oligonucleotide probes specific for the target.

Advantageously, the methods and kits of the invention provide for rapid detection of the amplification product (amplicon), which can be carried out with the naked eye generally within 3-20 minutes and requires no specific instruments or further sample manipulations. The operation is similar to that of a pH indicator. In the most simple embodiment of the invention, in fact, it is sufficient to add a small aliquot of the PCR reaction in a test tube containing a red solution and observe the result after approximately 3 minutes. If the solution remains red, this means that no amplicons, and therefore no targets, were present. On the contrary, if the PCR reaction contains the amplicon, the solution becomes violet/blue. This one-step one-tube procedure can also be performed by non-specialized personnel. If coupled with portable equipment and automated for PCR, it can be easily implemented in any context, even where there is no available laboratory equipment.

Further features and advantages of the invention will become apparent from the detailed description which follows, given purely by way of non-limiting example, with reference to the accompanying drawings, in which:
- Figure 1 is a diagram illustrating an asymmetric PCR amplification using a forward primer including at its 5' end a universal tag sequence (TAG) which is incorporated in the PCR amplification product;
- Figure 2 is a diagram illustrating a first embodiment of the detection method of the invention;
- Figure 3 is a diagram illustrating a second embodiment of the detection method of the invention;
- Figure 4 is a diagram illustrating a third embodiment of the detection method of the invention;
- Figure 5 is a diagram illustrating a fourth embodiment of the detection method of the invention;

The method of the invention is performed on a sample resulting from a nucleic acid amplification reaction capable of generating a single-stranded, or alternatively a double-stranded, amplification product (also designated as "amplicon"), in which a universal tag sequence is incorporated (TAG). As illustrated herein below, the universal tag sequence has a length of between 8 and 30 nucleotides. A single stranded amplicon incorporating a TAG is preferably obtained by asymmetric PCR; a double stranded amplicon incorporating a TAG is preferably obtained by standard PCR.

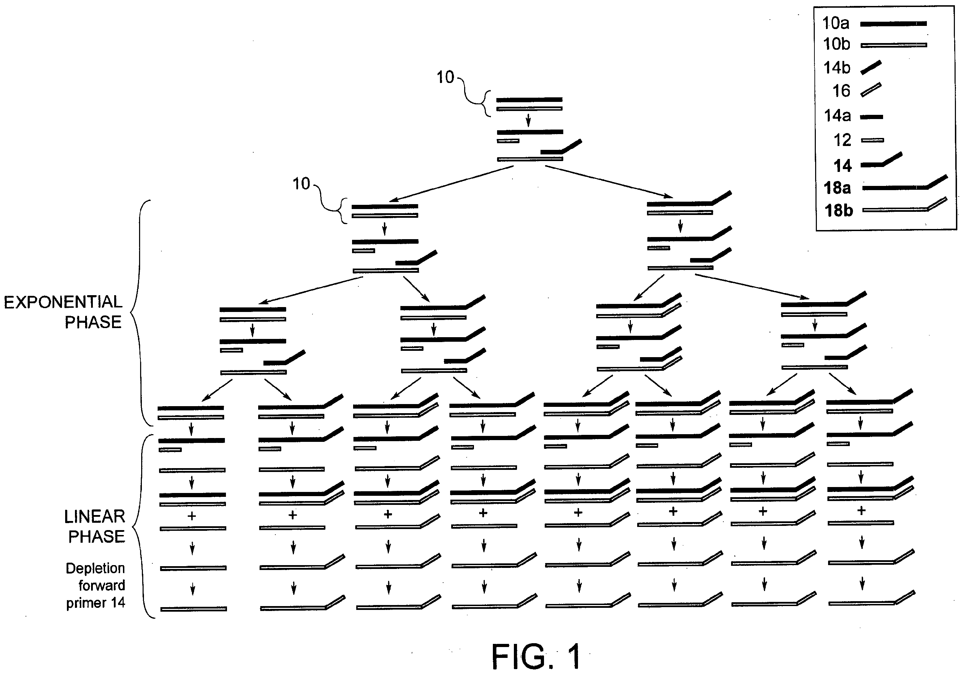

An asymmetric PCR reaction resulting in the incorporation of a universal tag sequence (TAG) in the amplification product is schematically illustrated in Figure 1.

10a and 10b, respectively, indicate the coding strand and the non-coding strand of the target nucleic acid 10, i.e. the nucleic acid region that is to be amplified and detected. The target DNA generally has a length of between approximately 30 and approximately 100 base pairs.

The amplification is performed by means of a reverse primer 12 that hybridizes to the coding strand 10a of the target 10 and a forward primer 14 that hybridizes to the non-coding strand 10b of the target 10. The forward primer 14 comprises a region binding the target 14a, consisting of a nucleic acid sequence designed to hybridize with the non-coding strand 10b, and a region 14b that is complementary to a universal tag sequence 16 having a length of between 8 and 30 nucleotides, the said region 14b not being capable of hybridizing to the target. The region 14b is located at the 5' end of the forward primer 14. As shown in Figure 1, in the course of the amplification reaction, the region 14b is incorporated in the amplification product 18a of the target nucleic acid 10, and the universal tag sequence 16 (which is complementary to region 14b) is incorporated in the amplification product 18b complementary to the amplification product 18a. Since in an asymmetric PCR the reverse primer 12 is used in excess over the forward primer 14 (the molar ratio of forward:reverse being generally between 1:5 and 1:1000, preferably between 1:20 and 1:50), in the last amplification cycles the forward primer 14 is depleted and the amplification is not exponential anymore. Consequently, at the end of the asymmetric PCR reaction, an accumulation of amplification product 18b occurs, which includes the universal tag sequence 16 at one of its ends. Figure 1 shows that the universal tag sequence 16 is not present at the beginning of the amplification reaction and is not synthesized in the absence of target nucleic acid 10. It therefore represents a universal indicator of the presence of the target 10 in the sample.

After the amplification reaction, the universal tag sequence 16 is detected by means of a method that constitutes the object of the present invention.

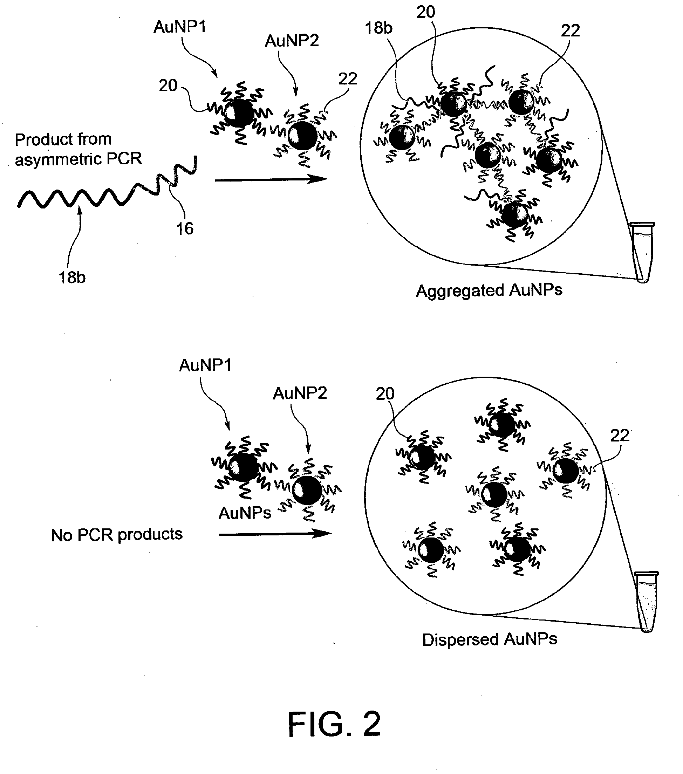

Figure 2 shows a first embodiment of the detection method of the invention, wherein the detection is carried out via a first colloidal gold nanoparticle AuNP-1 functionalized with a first oligonucleotide probe 20 and a second colloidal gold nanoparticle AuNP-2 functionalized with a second oligonucleotide probe 22. The first oligonucleotide probe 20 is complementary to a first portion of the universal tag sequence 16 and the second oligonucleotide probe 22 is complementary to a second portion of the universal tag sequence 16. To detect the success of the amplification colorimetrically, a premixed solution of the AuNP-1 and AuNP-2 colloidal gold nanoparticles is added to one aliquot of the sample subjected to asymmetric PCR illustrated in Figure 1. In the presence of target nucleic acid 10, the amplification reaction leads to the synthesis of the amplification product 18b including the universal tag sequence 16. This causes the cross-linking of the AuNP-1 and AuNP-2 colloidal gold nanoparticles via the respective oligonucleotide probes 20 and 22 that hybridize to the respective portions of the universal tag sequence 16. The end result is a change in colour of the solution from red to violet/blue. On the contrary, in the absence of target nucleic acid 10, the AuNP-1 and AuNP-2 colloidal gold nanoparticles do not cross-link because the amplification product 18b including the universal tag sequence 16 does not form and, consequently, variation in the colour of the solution is not observed. This embodiment advantageously allows to detect amplification of relatively short targets in an extremely short period of time (about 2-3 minutes).

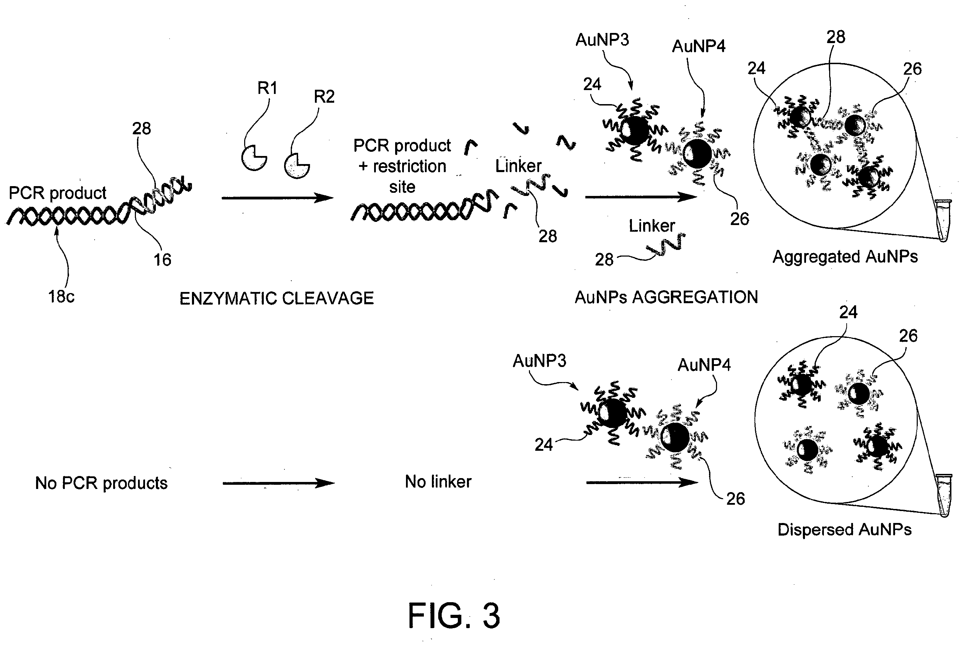

Figure 3 shows a second embodiment of the detection method of the invention, wherein the detection is carried out via a first colloidal gold nanoparticle AuNP-3 functionalized with a first oligonucleotide probe 24 and a second colloidal gold nanoparticle AuNP-4 functionalized with a second oligonucleotide probe 26. In this embodiment, the first oligonucleotide probe 24 is complementary to a first portion of a linker sequence 28 and the second oligonucleotide probe 26 is complementary to a second portion of the linker sequence 28. The linker sequence 28 is generated by enzymatic digestion of the universal tag sequence 16, which according to this embodiment is designed in such a way as to include a first restriction site, and optionally a second restriction site (not shown in Figure 3). The first restriction site is located at the proximal end of the universal tag sequence 16 and is able to be cleaved by a first restriction enzyme R1. The second optional restriction site is located at the distal end of the universal tag sequence 16 and is able to be cleaved by a second optional restriction enzyme R2. To detect the successful amplification of the target nucleic acid sequence colorimetrically, in a first step, the restriction enzyme or enzymes R1, R2 required to generate the linker sequence 28 and, in a second step, a premixed solution of the AuNP-3 and AuNP-4 colloidal gold nanoparticles are added to an aliquot of a sample subjected to symmetrical PCR. In the presence of target nucleic acid, the amplification reaction leads to the synthesis of a double-stranded amplification product 18c including the universal tag sequence 16, also in the double-stranded form. The latter is digested by the first and optionally the second restriction enzyme R1, R2 to give the single-stranded linker sequence 28, which in turn causes the cross-linking of the AuNP-3 and AuNP-4 nanoparticles via the respective oligonucleotide probes 24 and 26. The end result is a change in colour of the solution from red to violet/blue. On the contrary, in the absence of target nucleic acid, the AuNP-3 and AuNP-4 colloidal gold nanoparticles do not cross-link because the double-stranded amplification product 18c including the universal tag sequence 16 does not form and, consequently, the single-stranded linker sequence 28 does not form. Thus, no change in colour of the solution is observed. This embodiment advantageously allows for the detection of an amplification product of any length. It also allows for the detection of amplification products from symmetrical PCR reactions. At the end of the PCR reaction a brief digestion with the restriction enzyme or enzymes is carried out on small aliquots of the PCR product. The selected restriction enzyme or enzymes are high fidelity and fast enzymes, which allow for a complete digestion in about 10 minutes. In addition, they are chosen so as not to be affected by the components of the PCR buffer. Since this embodiment is particularly indicated for cloning procedures, the restriction enzymes are preferably selected from among the most widely used in these procedures, so that the user can directly use the digested PCR product after the detection procedure. For example, restriction enzymes that can be used in this application are: XhoI, EcoRI, SalI, XbaI, DraIII, KpnI, PstI, NheI, AgeI.

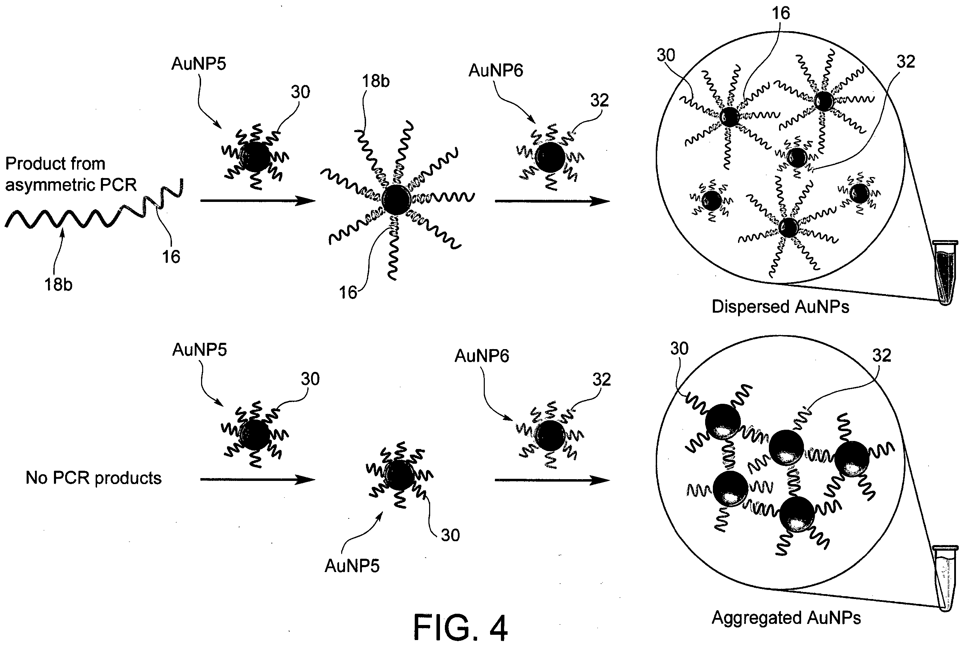

Figure 4 shows a third embodiment of the detection method of the invention, wherein the detection is carried out via a first colloidal gold nanoparticle AuNP-5 functionalized with a first oligonucleotide probe 30 and a second colloidal gold nanoparticle AuNP-6 functionalized with a second oligonucleotide probe 32. The first oligonucleotide probe 30 is complementary to a first portion of the universal tag sequence 16 and the second oligonucleotide probe 32 is complementary to the first oligonucleotide probe 30. To detect the success of the amplification colorimetrically, a solution of the AuNP-5 colloidal gold nanoparticles and then a solution of the AuNP-6 colloidal gold nanoparticles are added to an aliquot of the sample subjected to asymmetric PCR illustrated in Figure 1. In the presence of target nucleic acid 10, the amplification reaction leads to the synthesis of the amplification product 18b including the universal tag sequence 16, which hybridizes with the oligonucleotide probe 30 of the AuNP-5 nanoparticles, forming a structure stabilized by steric effects. The subsequent hybridization with the oligonucleotide probe 32 of the AuNP-6 particles is thus inhibited. Therefore, in the presence of the amplification product 18b, the solution remains red. On the contrary, in the absence of the amplification product 18b, the AuNP-5 and AuNP-6 nanoparticles aggregate, thanks to the hybridization of the oligonucleotide probe 30, with the complementary oligonucleotide probe 32 and therefore a change in colour can be observed. This embodiment advantageously allows for the detection of target nucleic acid sequences of any length with a very fast procedure.

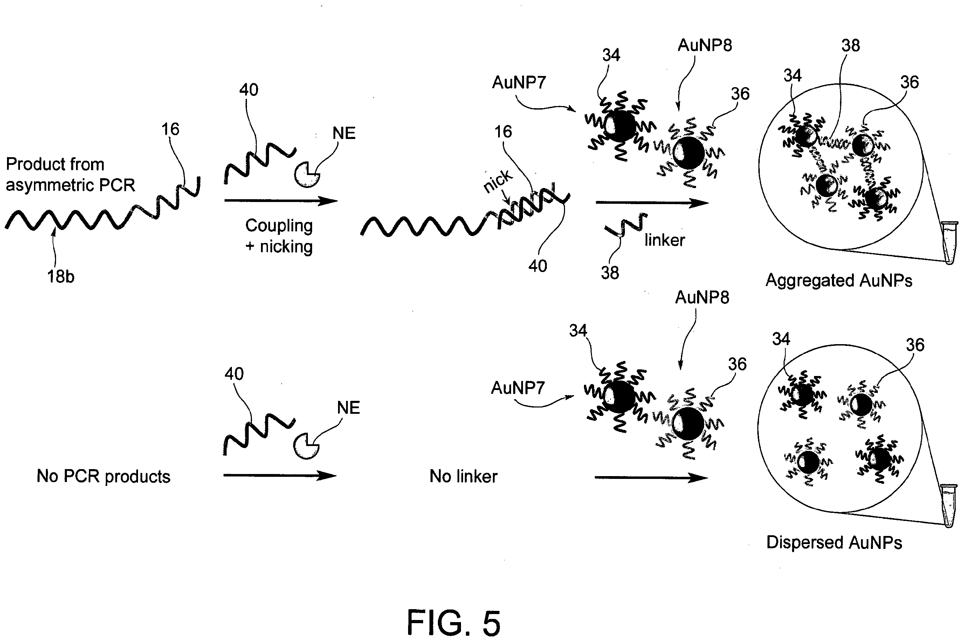

Figure 5 shows a fourth embodiment of the detection method of the invention, wherein the detection is carried out via a first colloidal gold nanoparticle AuNP-7 functionalized with a first oligonucleotide probe 34 and a second colloidal gold nanoparticle AuNP-8 functionalized with a second oligonucleotide probe 36. The first oligonucleotide probe 34 is complementary to a first portion of a linker sequence 38 and the second oligonucleotide probe 36 is complementary to a second portion of the linker sequence 38. The linker sequence 38 is generated by enzymatic digestion of the universal tag sequence 16, which according to this embodiment is designed in such a way as to include a restriction site for a nicking endonuclease NE. To detect the successful amplification of the target nucleic acid sequence colorimetrically, in a first step, the nicking endonuclease NE and a single-stranded sequence 40 complementary to a portion of the universal tag sequence 16 are added to an aliquot of a sample subjected to asymmetric PCR. In a second step, a premixed solution of the AuNP-7 and AuNP-8 colloidal gold nanoparticles is added. In the presence of target nucleic acid, the amplification reaction leads to the synthesis of the single-stranded amplification product 18b including the universal tag sequence 16. The complementary sequence 40 hybridizes to the universal tag sequence 16, forming a partially single-stranded and partially double-stranded nucleic acid hybrid, which is cut by the nicking endonuclease NE, thus generating the linker sequence 38. The linker sequence 38 causes the cross-linking of the AuNP-7 and AuNP-8 nanoparticles via the respective oligonucleotide probes 34 and 36. The end result is a change in colour of the solution from red to violet/blue. On the contrary, in the absence of target nucleic acid, the AuNP-7 and AuNP-8 colloidal gold nanoparticles do not cross-link because the amplification product 18b including the universal tag sequence 16 does not form and, consequently, the single-stranded linker sequence 38 does not form. Thus, no change in colour of the solution is observed. This embodiment allows for the detection of target nucleic acid sequences of any length.

In the above-described embodiments, suitable to be used for the detection of products from asymmetric or symmetric PCR, the PCR negative control sample also acts as a control for the detection method, as it contains all the reaction constituents with the only exception of the universal tag sequence 16.
Importantly, all the embodiments of the detection method of the invention employ a universal detector, i.e., the universal tag sequence 16, and are therefore also suitable for use in multi-well formats for simultaneous detection of a plurality of samples, even if they contain different target sequences. In the case of detection of different target sequences, as a preliminary control only the first time that the amplification is performed, it is sufficient to check on a gel that the amplification product is of the expected length and that there are no unspecific products.

The size (diameter) of the AuNP colloidal gold particles used in the detection methods according to the invention is generally comprised between 1 nm and 500 nm, preferably between 15 nm and 80 nm. Their density of functionalization with the oligonucleotide probes is preferably comprised between 2x10⁻⁴ and 2x10⁻¹ /nm², more preferably between 1x10⁻³/nm² and 8x10⁻²/nm².

In some embodiments, the oligonucleotide probes may include a spacer sequence that is not complementary to the universal tag sequence 16 and that serves to optimize the efficiency of the reaction. Preferably, the total length of the oligonucleotide probes ranges from 15 nucleotides (nt) to 80 nt, preferably from 15 nt to 40 nt. The length of the universal tag sequence 16 ranges from 8 nt to 30 nt.

A preferred minimum length is 8, 9 or 10 nucleotides.
It should be noted that the limited length of the tag, which is shorter than the typical short amplification product of a PCR (amplicon), causes a significant and advantageous increase in the sensitivity of the assay. The use of short universal tag sequences also allows for a more rapid colour change, which is critical for rapid and accurate detection to the naked eye.

It should also be noted that, although in principle colloidal gold particles functionalized with oligonucleotides complementary to respective regions of the target could hybridize at internal positions of the amplicon (in order to reduce the inter-particle distance and therefore maximize plasmon coupling), in practice this is not feasible because of the steric hindrance of the terminal regions of the amplicon. The free ends of the amplicon interfere with the hybridization of the AuNPs in the internal regions of the amplicon.

Preferred examples of the universal tag sequence 16 are:
TAAACTCTGATGTA (SEQ ID NO:1)
AAACTCTGATGT (SEQ ID NO:2)
AACTCTGATG (SEQ ID NO:3)
ACTCTGATG

Preferred examples of the first oligonucleotide probe are:
5' TTTTTATCATCATACATCA 3' (SEQ ID NO:4);
5' TTTTTATCATCTGTACCTG 3' (SEQ ID NO:5);
5' ACATCAGAGTCAACATTTTTTTTTT 3' (SEQ ID NO:6).

Preferred examples of the second oligonucleotide probe are:
5' GAGTTTACCAAGTATTTTTTTTTT 3' (SEQ ID NO:7);
5' GTGAATTACAAGTATTTTT 3' (SEQ ID NO:8);
5' TGTTGACTCTTTTTTTTTTT 3' (SEQ ID NO:9).

In the experimental section that follows, which is provided by way of illustration only, detection experiments are described which were carried out using the beta-actin gene as the target nucleic acid sequence. For the embodiment shown in Figure 2, an asymmetric PCR was performed with a primer pair that amplifies a relatively short target of 35 nucleotides. The reaction product was preliminarily analyzed by electrophoresis to confirm the presence of bands of the expected size. A small aliquot of the reaction product was then added to appropriately functionalized colloidal gold nanoparticles. For the embodiment shown in Figure 3, a symmetric PCR was carried out on a longer fragment of the beta-actin gene, and then an enzymatic digestion was performed for 10 minutes. A small aliquot of the enzymatic digestion reaction was then added to a mixture of appropriately functionalized colloidal gold nanoparticles. For the embodiment shown in Figure 4, an asymmetric PCR was performed and a first type of appropriately functionalized colloidal gold nanoparticles, and then a second type of gold nanoparticles functionalized with a complementary oligonucleotide, were added to a small aliquot of the reaction product. In all cases, the amplification reaction product could be detected with the naked eye in a few minutes.

### Examples

### Example 1

An asymmetric PCR was carried out using the forward primer: ACATCAGAGTTTCCAGCACAATGAAGATCA (SEQ ID NO:10) and the reverse primer: AGGAAAGACACCCACCT (SEQ ID NO:11).

These primers amplify a portion of 35 nucleotides of the gene encoding beta actin.

The reaction mixture contained 500 nM reverse primer, 25 nM forward primer, 1 µl of Taq polymerase, 0.2 mM dNTP mix, IX Taq reaction buffer, 2.5 mM MgCl₂ and 100 ng of genomic DNA extracted from HeLa cell cultures, in a total volume of 50 µl. A sample designated as the negative control was prepared in the same way, with the exception of the genomic DNA, which was absent in the negative control. After 35 rounds of PCR, an aliquot of each reaction was run on an 18% native polyacrylamide gel to verify the formation of the single-stranded amplification product. After that, an aliquot of the asymmetric PCR reaction (from 5 to 10 µl) was added to a 1:1 mixture of colloidal gold nanoparticles functionalized with a first and a second oligonucleotide probe respectively (named AuNPs-1 and AuNPs-2 respectively). First oligonucleotide probe: 5' TTTTTATCATCATACATCA 3' (SEQ ID NO:4); second oligonucleotide probe: 5' GAGTTTACCAAGTATTTTTTTTTT 3' (SEQ ID NO:7). The AuNP-1 and AuNP-2 mixture had a final concentration of 1 nM. After the addition of the PCR product, in a few minutes a change in colour could be observed, which was caused by the cross-linking of the two types of nanoparticles on the target through the universal tag sequence.

### Example 2

A standard symmetric PCR was carried out using the following primers:
forward: 5' TTGGTACCTGGTGAATTCCTTCCCTCCTCAGATCATTG 3' (SEQ ID NO:12) and reverse: 5' GATCCACACGGAGTACTTG 3' (SEQ ID NO:13).

These primers amplify a portion of 52 nucleotides (nt) of the gene encoding beta actin.

The reaction mixture contained 500 nM reverse primer, 500 nM forward primer, 1 µl of Taq polymerase, 0.2 mM dNTP mix, IX Taq reaction buffer, 2.5 mM MgCl₂ and 100 ng of genomic DNA extracted from HeLa cell cultures, in a total volume of 50 µl. A sample designated as the negative control was prepared in the same way, with the exception of the genomic DNA, which was absent in the negative control. After 35 rounds of PCR, an aliquot of each reaction was run on an 18% denaturing polyacrylamide gel to verify the formation of the amplification product of the desired length. A small aliquot of the PCR reaction (from 5 to 10 µl) was mixed with 1 µl of EcoRI-HF and 1 µl of KpnI-HF, in the presence or absence of the specific enzyme buffer, and incubated for 10 minutes at 37°C. Thereafter, an aliquot (from 5 to 10 µl) of the enzymatic digestion reaction was added to a 1:1 mixture of colloidal gold nanoparticles functionalized with two separate oligonucleotide probes (referred to respectively as AuNP-3 and AuNP-4). AuNP-3 oligonucleotide probe: 5' TTTTTATCATCTGTACCTG 3' (SEQ ID NO:5); AuNP-4 oligonucleotide probe: 5' GTGAATTACAAGTATTTTT 3' (SEQ ID NO:8). The AuNP-3 and AuNP-4 mixture had a final concentration of 1 nM. After a few minutes from the addition of the PCR product, a change in colour could be observed, which was caused by the cross-linking of the two types of nanoparticles through the linker formed by digestion of the amplification product with the restriction enzymes.

### Example 3

An asymmetric PCR was carried out using the forward primer 5' ACATCAGAGTCACTTCCCTCCTCAGATCATTG 3' (SEQ ID NO:14) and the reverse primer 5' GATCCACACGGAGTACTTG 3' (SEQ ID NO:15).

These primers amplify a portion of 52 nt of the gene encoding beta actin.

The reaction mixture contained 500 nM reverse primer, 25 nM forward primer, 1 µl of Taq polymerase, 0.2 mM dNTP mix, IX Taq reaction buffer, 2.5 mM MgCl₂ and 100 ng of genomic DNA extracted from HeLa cell cultures, in a total volume of 50 µl. A sample designated as the negative control was prepared in the same way, with the exception of the genomic DNA, which was absent in the negative control. After 35 rounds of PCR, one aliquot of each reaction was run on an 18% native polyacrylamide gel to verify the formation of the single-stranded amplification product. Thereafter, one aliquot (from 5 to 10 µl) of the asymmetric PCR reaction was added to AuNP-5 nanoparticles functionalized with the oligo probe 5' ACATCAGAGTCAACATTTTTTTTTT 3' (SEQ ID NO:6) and incubated for 10 minutes. Then, AuNP-6 nanoparticles functionalized with the oligo probe 5' TGTTGACTCTTTTTTTTTTT 3' (SEQ ID NO:9) were added to the mixture. The final concentration of AuNP-5 and AuNP-6 was 1 nM. After a few minutes a change in colour could be observed, which in this case indicated the absence of the target sequence (thus, it was only seen in the negative control).

## Claims

1. A method for detecting the amplification of a target nucleic acid sequence (10) in a sample resulting from a nucleic acid amplification reaction capable of generating a single-stranded amplification product (18b) comprising at one end a universal tag sequence (16) having a length of between 8 and 30 nucleotides, the method being **characterized in that** it comprises the steps of:
- adding to said sample colloidal gold nanoparticles (AuNP1, AuNP3, AuNP5, AuNP7) functionalized with a first oligonucleotide probe (20, 24, 30, 34) and colloidal gold nanoparticles (AuNP2, AuNP4, AuNP6, AuNP8) functionalized with a second oligonucleotide probe (22, 26, 32, 36), wherein said first oligonucleotide probe is at least partially complementary to a first portion of said universal tag sequence (16) and said second oligonucleotide probe is at least partially complementary to a second portion of said universal tag sequence (16) or said second oligonucleotide probe is at least partially complementary to said first oligonucleotide probe;
- detecting the possible colour change of the sample as a result of the addition of said functionalized colloidal gold nanoparticles (AuNP1, AuNP3, AuNP5, AuNP7; AuNP2, AuNP4, AuNP6, AuNP8),
wherein, when the second oligonucleotide probe is at least partially complementary to the second portion of the universal tag sequence, the colour change of the sample is indicative of the successful amplification of the target nucleic acid sequence, whereas when the second oligonucleotide probe is at least partially complementary to the first oligonucleotide probe, the absence of colour change of the sample is indicative of the successful amplification of the target nucleic acid sequence.

2. A method for detecting the amplification of a target nucleic acid sequence (10) in a sample resulting from a nucleic acid amplification reaction capable of generating a single-stranded amplification product (18b) comprising at one end a universal tag sequence (16) having a length of between 8 and 30 nucleotides, said universal tag sequence (16) including a nicking endonuclease (NE) recognition site, the method being **characterized in that** it comprises the steps of:
- adding to said sample a single-stranded oligonucleotide (40) complementary to the universal tag sequence (16);
- incubating under suitable conditions so that hybridization occurs with the single-stranded amplification product (18b) possibly present in the sample, whereby a hybrid partially single-stranded and partially double-stranded nucleic acid is generated;
- after said incubation, incubating the sample with said nicking endonuclease (NE), whereby a single-stranded linker sequence (38) consisting of at least one portion of the universal tag sequence (16) is released from said hybrid partially single-stranded and partially double-stranded nucleic acid possibly generated;
- adding to the sample colloidal gold nanoparticles (AuNP7) functionalized with a first oligonucleotide probe (34) and colloidal gold nanoparticles (AuNP8) functionalized with a second oligonucleotide probe (36), wherein said first and second oligonucleotide probes (34, 36) are complementary to respective portions of said linker sequence (38);
- detecting the possible colour change of the sample as a result of the addition of said functionalized colloidal gold nanoparticles (AuNP7, AuNP8),
wherein a colour change of the sample is indicative of the successful amplification of the target nucleic acid sequence.

3. A method for detecting the amplification of a target nucleic acid sequence (10) in a sample resulting from a nucleic acid amplification reaction capable of generating a double-stranded amplification product (18c) comprising at one end a universal tag sequence (16) having a length of between 8 and 30 nucleotides, said universal tag sequence (16) including a first restriction site and optionally a second restriction site, wherein said first restriction site is located at the proximal end of the universal tag sequence (16) and is able to be cleaved by a first restriction enzyme (R1), and wherein said second optional restriction site is located at the distal end of the universal tag sequence and is able to be cleaved by a second restriction enzyme (R2), the method being **characterized in that** it comprises the steps of:
- adding to said sample said first restriction enzyme (R1) and optionally said second restriction enzyme (R2);
- incubating under conditions suitable for an enzymatic digestion reaction brought about by said first restriction enzyme (R1) and optionally by said second restriction enzyme (R2) to occur, whereby a single-stranded linker sequence (28) consisting of at least one portion of the universal tag sequence (16) is released from the amplification product (18c) possibly present in the sample;
- adding to said sample colloidal gold nanoparticles (AuNP3) functionalized with a first oligonucleotide probe (24) and colloidal gold nanoparticles (AuNP4) functionalized with a second oligonucleotide probe (26), wherein said first and second oligonucleotide probes (24, 26) are at least partially complementary to respective portions of said linker sequence (28);
- detecting the possible colour change of the sample as a result of the addition of said functionalized colloidal gold nanoparticles (AuNP3, AuNP4),
wherein a colour change of the sample is indicative of the successful amplification of the target nucleic acid sequence.

4. The method according to claim 1 or 2, wherein the nucleic acid amplification reaction is an asymmetric PCR employing a forward primer (14) and a reverse primer (12), wherein the reverse primer (12) is in excess over the forward primer (14), the molar ratio between forward primer (14) and reverse primer (12) being preferably between 1:5 and 1:1000, more preferably between 1:20 and 1:50.

5. The method according to claim 3, wherein the nucleic acid amplification reaction is a symmetric PCR employing a forward primer (14) and a reverse primer (12).

6. The method according to any one of claims 1 to 5, wherein the length of each of said first and second oligonucleotide probes (20, 24, 30, 34; 22, 26, 32, 36) with which the colloidal gold particles are functionalized is between 5 and 80 nucleotides, preferably between 12 and 40 nucleotides.

7. The method according to any one of claims 1 to 6, wherein the universal tag sequence (16) included in the nucleic acid amplification reaction product is selected from the group consisting of TAA ACT CTG ATG TA (SEQ ID NO:1), AA ACT CTG ATG T (SEQ ID NO:2), A ACT CTG ATG (SEQ ID NO:3) and ACT CTG ATG.

8. The method according to any one of claims 1 to 7, wherein said colloidal gold nanoparticles have a particle size of between 1 and 500 nm, preferably between 15 and 80 nm.

9. The method according to any one of claims 1 to 8, wherein said colloidal gold nanoparticles are functionalized at a functionalization density of from 2x10⁻⁴/nm² to 2x10⁻¹/nm², preferably of from 1x10⁻³/nm² to 8x10⁻²/nm².

10. A kit for detecting the amplification of a target nucleic acid sequence (10) in a sample resulting from a nucleic acid amplification reaction capable of generating an amplification product (18b, 18c) including at one end a universal tag sequence (16) having a length of between 8 and 30 nucleotides, the kit being **characterized in that** it comprises:
- colloidal gold nanoparticles (AuNP1, AuNP3, AuNP5, AuNP7) functionalized with a first oligonucleotide probe (20, 24, 30, 34); and
- colloidal gold nanoparticles (AuNP2, AuNP4, AuNP6, AuNP8) functionalized with a second oligonucleotide probe (22, 26, 32, 36),
wherein said first oligonucleotide probe (20, 24, 30, 34) is at least partially complementary to a first portion of said universal tag sequence (16) and said second oligonucleotide probe (22, 26, 32, 36) is at least partially complementary to a second portion of said universal tag sequence (16) or said second oligonucleotide probe (22, 26, 32, 36) is at least partially complementary to said first oligonucleotide probe (20, 24, 30, 34),
**characterised in that** it also comprises a nicking endonuclease (NE) and a single-stranded oligonucleotide (40) complementary to the universal tag sequence (16), wherein the universal tag sequence (16) includes a recognition site for said nicking endonuclease (NE).

11. A kit for detecting the amplification of a target nucleic acid sequence (10) in a sample resulting from a nucleic acid amplification reaction capable of generating an amplification product (18b, 18c) including at one end a universal tag sequence (16) having a length of between 8 and 30 nucleotides, the kit being **characterized in that** it comprises:
- colloidal gold nanoparticles (AuNP1, AuNP3, AuNP5, AuNP7) functionalized with a first oligonucleotide probe (20, 24, 30, 34);
- colloidal gold nanoparticles (AuNP2, AuNP4, AuNP6, AuNP8) functionalized with a second oligonucleotide probe (22, 26, 32, 36);
wherein said first oligonucleotide probe (20, 24, 30, 34) is at least partially complementary to a first portion of said universal tag sequence (16) and said second oligonucleotide probe (22, 26, 32, 36) is at least partially complementary to a second portion of said universal tag sequence (16) or said second oligonucleotide probe (22, 26, 32, 36) is at least partially complementary to said first oligonucleotide probe (20, 24, 30, 34),
**characterised in that** it also comprises a first restriction enzyme (R1) designed to cleave the universal tag sequence (16) at a first restriction site, and optionally a second restriction enzyme (R2) designed to cleave the universal tag sequence (16) at a second restriction site, the universal tag sequence (16) including said first restriction site and optionally said second restriction site.

12. The kit according to claims 10 or 11, wherein the length of each of said first and second oligonucleotide probes (20, 24, 30, 34; 22, 26, 32, 36) with which the colloidal gold particles are functionalized is between 5 and 80 nucleotides, preferably between 15 and 40 nucleotides.

13. The kit according to any one of claims 10 to 12, wherein said colloidal gold nanoparticles have a particle size of between 1 and 500 nm, preferably between 15 and 80 nm.

14. The kit according to any one of claims 10 to 13, wherein said colloidal gold nanoparticles functionalized with a first oligonucleotide probe and said colloidal gold nanoparticles functionalized with a second oligonucleotide probe have a functionalization density of from 2x10⁻⁴ to 2x10⁻¹/nm², preferably from 1x10⁻³/nm² to 8x10⁻²/nm².

## Patentansprüche

1. Verfahren zum Nachweis der Amplifizierung einer Zielnukleinsäuresequenz (10) in einer Probe, die aus einer Nukleinsäureamplifizierungsreaktion resultiert, die in der Lage ist, ein einzelsträngiges Amplifikationsprodukt (18b) zu erzeugen, das an einem Ende eine universelle Markierungssequenz (16) mit einer Länge zwischen 8 und 30 Nukleotiden umfasst, wobei das Verfahren **dadurch gekennzeichnet ist, dass** es die folgenden Schritte umfasst:
- Zugeben von kolloidalen Goldnanopartikeln (AuNP1, AuNP3, AuNP5, AuNP7), die mit einer ersten Oligonukleotidsonde (20, 24, 30, 34) funktionalisiert sind, und von kolloidalen Goldnanopartikeln (AuNP2, AuNP4, AuNP6, AuNP8), die mit einer zweiten Oligonukleotidsonde funktionalisiert sind (22, 26, 32, 36), zu der Probe, wobei die erste Oligonukleotidsonde wenigstens zum Teil zu einem ersten Abschnitt der universellen Markierungssequenz (16) komplementär ist und die zweite Oligonukleotidsonde wenigstens zum Teil zu einem zweiten Abschnitt der universellen Markierungssequenz (16) komplementär ist oder die zweite Oligonukleotidsonde wenigstens zum Teil zu der ersten Oligonukleotidsonde komplementär ist;
- Erfassen der möglichen Farbveränderung der Probe infolge der Zugabe der funktionalisierten kolloidalen Goldnanopartikel (AuNP1, AuNP3, AuNP5, AuNP7; AuNP2, AuNP4, AuNP6, AuNP8),
wobei, wenn die zweite Oligonukleotidsonde wenigstens zum Teil zu dem zweiten Abschnitt der universellen Markierungssequenz komplementär ist, die Farbveränderung der Probe eine erfolgreiche Amplifizierung der Zielnukleinsäuresequenz anzeigt, wohingegen, wenn die zweite Oligonukleotidsonde wenigstens zum Teil zu der ersten Oligonukleotidsonde komplementär ist, das Fehlen einer Farbveränderung der Probe die erfolgreiche Amplifizierung der Zielnukleinsäuresequenz anzeigt.

2. Verfahren zum Nachweis der Amplifizierung einer Zielnukleinsäuresequenz (10) in einer Probe, die aus einer Nukleinsäureamplifizierungsreaktion resultiert, die in der Lage ist, ein einzelsträngiges Amplifikationsprodukt (18b) zu erzeugen, das an einem Ende eine universelle Markierungssequenz (16) mit einer Länge zwischen 8 und 30 Nukleotiden umfasst, wobei die universelle Markierungssequenz (16) eine Erkennungsstelle für Nicking-Endonuklease (NE) enthält, wobei das Verfahren **dadurch gekennzeichnet ist, dass** es die folgenden Schritte umfasst:
- Zugeben eines einzelsträngigen Oligonukleotids (40), das zu der universellen Markierungssequenz (16) komplementär ist, zu der Probe;
- Inkubieren unter geeigneten Bedingungen, so dass eine Hybridisierung mit dem möglicherweise in der Probe vorhandenen einzelsträngigen Amplifizierungsprodukt (18b) erfolgt, wodurch eine teilweise einzelsträngige und teilweise doppelsträngige Hybridnukleinsäure erzeugt wird;
- nach der Inkubation Inkubieren der Probe mit der Nicking-Endonuklease (NE), wobei eine einzelsträngige Linkersequenz (38), die aus wenigstens einem Abschnitt der universellen Markierungssequenz (16) besteht, aus der möglicherweise erzeugten teilweise einzelsträngigen und teilweise doppelsträngigen Hybridnukleinsäure freigesetzt wird;
- Zugeben von kolloidalen Goldnanopartikeln (AuNP7), die mit einer ersten Oligonukleotidsonde (34) funktionalisiert sind, und von kolloidalen Goldnanopartikeln (AuNP8), die mit einer zweiten Oligonukleotidsonde (36) funktionalisiert sind, zu der Probe, wobei die erste und die zweite Oligonukleotidsonde (34, 36) zu jeweiligen Abschnitten der Linkersequenz (38) komplementär sind;
- Erfassen der möglichen Farbveränderung der Probe infolge der Zugabe der funktionalisierten kolloidalen Goldnanopartikel (AuNP7, AuNP8),
wobei eine Farbveränderung der Probe die erfolgreiche Amplifizierung der Zielnukleinsäuresequenz anzeigt.

3. Verfahren zum Nachweis der Amplifizierung einer Zielnukleinsäuresequenz (10) in einer Probe, die aus einer Nukleinsäureamplifizierungsreaktion resultiert, die in der Lage ist, ein doppelsträngiges Amplifizierungsprodukt (18c) zu erzeugen, das an einem Ende eine universelle Markierungssequenz (16) mit einer Länge zwischen 8 und 30 Nukleotiden umfasst, wobei die universelle Markierungssequenz (16) eine erste Restriktionsstelle und gegebenenfalls eine zweite Restriktionsstelle enthält, wobei sich die erste Restriktionsstelle am proximalen Ende der universellen Markierungssequenz (16) befindet und durch ein erstes Restriktionsenzym (R1) gespalten werden kann, und wobei sich die zweite gegebenenfalls vorhandene Restriktionsstelle am distalen Ende der universellen Markierungssequenz befindet und durch ein zweites Restriktionsenzym (R2) gespalten werden kann, wobei das Verfahren **dadurch gekennzeichnet ist, dass** es die folgenden Schritte umfasst:
- Zugeben des ersten Restriktionsenzyms (R1) und gegebenenfalls des zweiten Restriktionsenzyms (R2) zu der Probe;
- Inkubieren unter Bedingungen, die für eine enzymatische Aufschlussreaktion geeignet sind, die durch das erste Restriktionsenzym (R1) und gegebenenfalls durch das zweite Restriktionsenzym (R2) hervorgerufen wird, wodurch eine einzelsträngige Linkersequenz (28), die aus wenigstens einem Abschnitt der universellen Markierungssequenz (16) besteht, aus dem möglicherweise in der Probe vorhandenen Amplifikationsprodukt (18c) freigesetzt wird;
- Zugeben von kolloidalen Goldnanopartikeln (AuNP3), die mit einer ersten Oligonukleotidsonde (24) funktionalisiert sind, und von kolloidalen Goldnanopartikeln (AuNP4), die mit einer zweiten Oligonukleotidsonde (26) funktionalisiert sind, zu der Probe, wobei die erste und die zweite Oligonukleotidsonde (24, 26) wenigstens zum Teil zu jeweiligen Abschnitten der Linkersequenz (28) komplementär sind;
- Erfassen der möglichen Farbveränderung der Probe infolge der Zugabe der funktionalisierten kolloidalen Goldnanopartikel (AuNP3, AuNP4),
wobei eine Farbveränderung der Probe die erfolgreiche Amplifizierung der Zielnukleinsäuresequenz anzeigt.

4. Verfahren nach Anspruch 1 oder 2, wobei die Nukleinsäureamplifizierungsreaktion eine asymmetrische PCR ist, die einen Vorwärtsprimer (14) und einen Rückwärtsprimer (12) verwendet, wobei der Rückwärtsprimer (12) gegenüber dem Vorwärtsprimer (14) im Überschuss vorliegt, wobei das Molverhältnis zwischen dem Vorwärtsprimer (14) und dem Rückwärtsprimer (12) vorzugsweise zwischen 1:5 und 1:1000 liegt, insbesondere zwischen 1:20 und 1:50.

5. Verfahren nach Anspruch 3, wobei die Nukleinsäureamplifizierungsreaktion eine symmetrische PCR ist, die einen Vorwärtsprimer (14) und einen Rückwärtsprimer (12) verwendet.

6. Verfahren nach einem der Ansprüche 1 bis 5, wobei die Länge der ersten und der zweiten Oligonukleotidsonde (20, 24, 30, 34; 22, 26, 32, 36), mit denen die kolloidalen Goldpartikel funktionalisiert sind, jeweils zwischen 5 und 80 Nukleotiden, vorzugsweise zwischen 12 und 40 Nukleotiden ist.

7. Verfahren nach einem der Ansprüche 1 bis 6, wobei die universelle Markierungssequenz (16), die in dem Reaktionsprodukt der Nukleinsäureamplifizierungsreaktion enthalten ist, ausgewählt ist aus der Gruppe bestehend aus TAA ACT CTG ATG TA (SEQ ID Nr:1), AA ACT CTG ATG T (SEQ ID Nr:2), A ACT CTG ATG (SEQ ID Nr:3) und ACT CTG ATG.

8. Verfahren nach einem der Ansprüche 1 bis 7, wobei die kolloidalen Goldnanopartikel eine Partikelgröße zwischen 1 und 500 nm, vorzugsweise zwischen 15 und 80 nm aufweisen.

9. Verfahren nach einem der Ansprüche 1 bis 8, wobei die kolloidalen Goldnanopartikel mit einer Funktionalisierungsdichte von 2x10⁻⁴/nm² bis 2x10⁻¹/nm², vorzugsweise von 1x10⁻³/nm² bis 8x10⁻²/nm² funktionalisiert sind.

10. Kit zum Nachweis der Amplifizierung einer Zielnukleinsäuresequenz (10) in einer Probe, die aus einer Nukleinsäureamplifizierungsreaktion resultiert, die in der Lage ist, ein Amplifikationsprodukt (18b, 18c) zu erzeugen, das an einem Ende eine universelle Markierungssequenz (16) mit einer Länge zwischen 8 und 30 Nukleotiden enthält, wobei der Kit **dadurch gekennzeichnet ist, dass** er Folgendes umfasst:
- mit einer ersten Oligonukleotidsonde (20, 24, 30, 34) funktionalisierte kolloidale Goldnanopartikel (AuNP1, AuNP3, AuNP5, AuNP7); und
- mit einer zweiten Oligonukleotidsonde (22, 26, 32, 36) funktionalisierte kolloidale Goldnanopartikel (AuNP2, AuNP4, AuNP6, AuNP8),
wobei die erste Oligonukleotidsonde (20, 24, 30, 34) wenigstens zum Teil zu einem ersten Abschnitt der universellen Markierungssequenz (16) komplementär ist und die zweite Oligonukleotidsonde (22, 26, 32, 36) wenigstens zum Teil zu einem zweiten Abschnitt der universellen Markierungssequenz (16) komplementär ist oder die zweite Oligonukleotidsonde (22, 26, 32, 36) wenigstens zum Teil zu der ersten Oligonukleotidsonde (20, 24, 30, 34) komplementär ist,
**dadurch gekennzeichnet, dass** er auch eine Nicking-Endonuklease (NE) sowie ein einzelsträngiges Oligonukleotid (40) umfasst, das zu der universellen Markierungssequenz (16) komplementär ist, wobei die universelle Markierungssequenz (16) eine Erkennungsstelle für die Nicking-Endonuklease (NE) enthält.

11. Kit zum Nachweis der Amplifizierung einer Zielnukleinsäuresequenz (10) in einer Probe, die aus einer Nukleinsäureamplifizierungsreaktion resultiert, die in der Lage ist, ein Amplifikationsprodukt (18b, 18c) zu erzeugen, das an einem Ende eine universelle Markierungssequenz (16) mit einer Länge zwischen 8 und 30 Nukleotiden enthält, wobei der Kit **dadurch gekennzeichnet ist, dass** er Folgendes umfasst:
- mit einer ersten Oligonukleotidsonde (20, 24, 30, 34) funktionalisierte kolloidale Goldnanopartikel (AuNP1, AuNP3, AuNP5, AuNP7);
- mit einer zweiten Oligonukleotidsonde (22, 26, 32, 36) funktionalisierte kolloidale Goldnanopartikel (AuNP2, AuNP4, AuNP6, AuNP8);
wobei die erste Oligonukleotidsonde (20, 24, 30, 34) wenigstens zum Teil zu einem ersten Abschnitt der universellen Markierungssequenz (16) komplementär ist und die zweite Oligonukleotidsonde (22, 26, 32, 36) wenigstens zum Teil zu einem zweiten Abschnitt der universellen Markierungssequenz (16) komplementär ist oder die zweite Oligonukleotidsonde (22, 26, 32, 36) wenigstens zum Teil zu der ersten Oligonukleotidsonde (20, 24, 30, 34) komplementär ist,
**dadurch gekennzeichnet, dass** er auch ein erstes Restriktionsenzym (R1) umfasst, das zur Spaltung der universellen Markierungssequenz (16) an einer ersten Restriktionsstelle ausgelegt ist, und gegebenenfalls ein zweites Restriktionsenzym (R2), das zur Spaltung der universellen Markierungssequenz (16) an einer zweiten Restriktionsstelle ausgelegt ist, wobei die universelle Markierungssequenz (16) die erste Restriktionsstelle und gegebenenfalls die zweite Restriktionsstelle enthält.

12. Kit nach Anspruch 10 oder 11, wobei die Länge der ersten und der zweiten Oligonukleotidsonde (20, 24, 30, 34; 22, 26, 32, 36), mit denen die kolloidalen Goldpartikel funktionalisiert sind, jeweils zwischen 5 und 80 Nukleotiden, vorzugsweise zwischen 15 und 40 Nukleotiden ist.

13. Kit nach einem der Ansprüche 10 bis 12, wobei die kolloidalen Goldnanopartikel eine Partikelgröße zwischen 1 und 500 nm, vorzugsweise zwischen 15 und 80 nm aufweisen.

14. Kit nach einem der Ansprüche 10 bis 13, wobei die mit einer ersten Oligonukleotidsonde funktionalisierten kolloidalen Goldnanopartikel und die mit einer zweiten Oligonukleotidsonde funktionalisierten kolloidalen Goldnanopartikel eine Funktionalisierungsdichte von 2x10⁻⁴/nm² bis 2x10⁻¹/nm², vorzugsweise von 1x10⁻³/nm² bis 8x10⁻²/nm² aufweisen.

## Revendications

1. Procédé de détection de l'amplification d'une séquence d'acide nucléique cible (10) dans un échantillon résultant d'une réaction d'amplification d'acide nucléique capable de générer un produit d'amplification simple brin (18b) comprenant à une extrémité une séquence d'étiquette universelle (16) ayant une longueur comprise entre 8 et 30 nucléotides, le procédé étant **caractérisé en ce qu'**il comprend les étapes de :
- ajout audit échantillon de nanoparticules d'or colloïdal (AuNP1, AuNP3, AuNP5, AuNP7) fonctionnalisées avec une première sonde oligonucléotidique (20, 24, 30, 34) et de nanoparticules d'or colloïdal (AuNP2, AuNP4, AuNP6, AuNP8) fonctionnalisées avec une seconde sonde oligonucléotidique (22, 26, 32, 36), dans lequel ladite première sonde oligonucléotidique est au moins partiellement complémentaire à une première partie de ladite séquence d'étiquette universelle (16) et ladite seconde sonde oligonucléotidique est au moins partiellement complémentaire à une seconde partie de ladite séquence d'étiquette universelle (16) ou ladite seconde sonde oligonucléotidique est au moins partiellement complémentaire à ladite première sonde oligonucléotidique ;
- détection du possible changement de couleur de l'échantillon à la suite de l'ajout desdites nanoparticules d'or colloïdal fonctionnalisées (AuNP1, AuNP3, AuNP5, AuNP7 ; AuNP2, AuNP4, AuNP6, AuNP8),
dans lequel, quand la seconde sonde oligonucléotidique est au moins partiellement complémentaire à la seconde partie de la séquence d'étiquette universelle, le changement de couleur de l'échantillon indique l'amplification réussie de la séquence d'acide nucléique cible, tandis que quand la seconde sonde oligonucléotidique est au moins partiellement complémentaire à la première sonde oligonucléotidique, l'absence de changement de couleur de l'échantillon indique l'amplification réussie de la séquence d'acide nucléique cible.

2. Procédé de détection de l'amplification d'une séquence d'acide nucléique cible (10) dans un échantillon résultant d'une réaction d'amplification d'acide nucléique capable de générer un produit d'amplification simple brin (18b) comprenant à une extrémité une séquence d'étiquette universelle (16) ayant une longueur comprise entre 8 et 30 nucléotides, ladite séquence d'étiquette universelle (16) comprenant un site de reconnaissance d'endonucléase de coupure (NE), le procédé étant **caractérisé en ce qu'**il comprend les étapes de :
- ajout audit échantillon d'un oligonucléotide simple brin (40) complémentaire à la séquence d'étiquette universelle (16) ;
- incubation dans des conditions appropriées pour qu'une hybridation se produise avec le produit d'amplification simple brin (18b) éventuellement présent dans l'échantillon, moyennant quoi un acide nucléique hybride partiellement simple brin et partiellement double brin est généré ;
- après ladite incubation, incubation de l'échantillon avec ladite endonucléase de coupure (NE), moyennant quoi une séquence de lieur simple brin (38) constituée d'au moins une partie de la séquence d'étiquette universelle (16) est libérée dudit acide nucléique hybride partiellement simple brin et partiellement double brin éventuellement généré ;
- ajout à l'échantillon de nanoparticules d'or colloïdal (AuNP7) fonctionnalisées avec une première sonde oligonucléotidique (34) et de nanoparticules d'or colloïdal (AuNP8) fonctionnalisées avec une seconde sonde oligonucléotidique (36), dans lequel lesdites première et seconde sondes oligonucléotidiques (34, 36) sont complémentaires à des parties respectives de ladite séquence de lieur (38) ;
- détection du possible changement de couleur de l'échantillon à la suite de l'ajout desdites nanoparticules d'or colloïdal fonctionnalisées (AuNP7, AuNP8),
dans lequel un changement de couleur de l'échantillon indique l'amplification réussie de la séquence d'acide nucléique cible.

3. Procédé de détection de l'amplification d'une séquence d'acide nucléique cible (10) dans un échantillon résultant d'une réaction d'amplification d'acide nucléique capable de générer un produit d'amplification double brin (18c) comprenant à une extrémité une séquence d'étiquette universelle (16) ayant une longueur comprise entre 8 et 30 nucléotides, ladite séquence d'étiquette universelle (16) comprenant un premier site de restriction et facultativement un second site de restriction, dans lequel ledit premier site de restriction est situé au niveau de l'extrémité proximale de la séquence d'étiquette universelle (16) et est susceptible d'être clivé par une première enzyme de restriction (R1), et dans lequel ledit second site de restriction facultatif est situé au niveau de l'extrémité distale de la séquence d'étiquette universelle et est susceptible d'être clivé par une seconde enzyme de restriction (R2), le procédé étant **caractérisé en ce qu'**il comprend les étapes de :
- ajout audit échantillon de ladite première enzyme de restriction (R1) et facultativement de ladite seconde enzyme de restriction (R2) ;
- incubation dans des conditions appropriées pour qu'une réaction de digestion enzymatique provoquée par ladite première enzyme de restriction (R1) et facultativement par ladite seconde enzyme de restriction (R2) se produise, moyennant quoi une séquence de lieur simple brin (28) constituée d'au moins une partie de la séquence d'étiquette universelle (16) est libérée du produit d'amplification (18c) éventuellement présent dans l'échantillon ;
- ajout audit échantillon de nanoparticules d'or colloïdal (AuNP3) fonctionnalisées avec une première sonde oligonucléotidique (24) et de nanoparticules d'or colloïdal (AuNP4) fonctionnalisées avec une seconde sonde oligonucléotidique (26), dans lequel lesdites première et seconde sondes oligonucléotidiques (24, 26) sont au moins partiellement complémentaires à des parties respectives de ladite séquence de lieur (28) ;
- détection du possible changement de couleur de l'échantillon à la suite de l'ajout desdites nanoparticules d'or colloïdal fonctionnalisées (AuNP3, AuNP4),
dans lequel un changement de couleur de l'échantillon indique l'amplification réussie de la séquence d'acide nucléique cible.

4. Procédé selon la revendication 1 ou 2, dans lequel la réaction d'amplification d'acide nucléique est une PCR asymétrique utilisant une amorce sens (14) et une amorce antisens (12), dans lequel l'amorce antisens (12) est en excès par rapport à l'amorce sens (14), le rapport molaire entre l'amorce sens (14) et l'amorce antisens (12) étant de préférence situé entre 1:5 et 1:1 000, de manière davantage préférée entre 1:20 et 1:50.

5. Procédé selon la revendication 3, dans lequel la réaction d'amplification d'acide nucléique est une PCR symétrique utilisant une amorce sens (14) et une amorce antisens (12).

6. Procédé selon l'une quelconque des revendications 1 à 5, dans lequel la longueur de chacune desdites première et seconde sondes oligonucléotidiques (20, 24, 30, 34 ; 22, 26, 32, 36) avec laquelle les particules d'or colloïdal sont fonctionnalisées est comprise entre 5 et 80 nucléotides, de préférence entre 12 et 40 nucléotides.

7. Procédé selon l'une quelconque des revendications 1 à 6, dans lequel la séquence d'étiquette universelle (16) incluse dans le produit de réaction d'amplification d'acide nucléique est sélectionnée dans le groupe constitué par TAA ACT CTG ATG TA (SEQ ID NO : 1), AA ACT CTG ATG T (SEQ ID NO : 2), A ACT CTG ATG (SEQ ID NO : 3) et ACT CTG ATG.

8. Procédé selon l'une quelconque des revendications 1 à 7, dans lequel lesdites nanoparticules d'or colloïdal présentent une taille particulaire comprise entre 1 et 500 nm, de préférence entre 15 et 80 nm.

9. Procédé selon l'une quelconque des revendications 1 à 8, dans lequel lesdites nanoparticules d'or colloïdal sont fonctionnalisées à une densité de fonctionnalisation de 2x10⁻⁴/nm² à 2x10⁻¹/nm², de préférence de 1x10⁻³/nm² à 8x10⁻²/nm².

10. Kit pour détecter l'amplification d'une séquence d'acide nucléique cible (10) dans un échantillon résultant d'une réaction d'amplification d'acide nucléique capable de générer un produit d'amplification (18b, 18c) comprenant à une extrémité une séquence d'étiquette universelle (16) ayant une longueur comprise entre 8 et 30 nucléotides, le kit étant **caractérisé en ce qu'**il comprend :
- des nanoparticules d'or colloïdal (AuNP1, AuNP3, AuNP5, AuNP7) fonctionnalisées avec une première sonde oligonucléotidique (20, 24, 30, 34) ; et
- des nanoparticules d'or colloïdal (AuNP2, AuNP4, AuNP6, AuNP8) fonctionnalisées avec une seconde sonde oligonucléotidique (22, 26, 32, 36),
dans lequel ladite première sonde oligonucléotidique (20, 24, 30, 34) est au moins partiellement complémentaire à une première partie de ladite séquence d'étiquette universelle (16) et ladite seconde sonde oligonucléotidique (22, 26, 32, 36) est au moins partiellement complémentaire à une seconde partie de ladite séquence d'étiquette universelle (16) ou ladite seconde sonde oligonucléotidique (22, 26, 32, 36) est au moins partiellement complémentaire à ladite première sonde oligonucléotidique (20, 24, 30, 34),
**caractérisé en ce qu'**il comprend également une endonucléase de coupure (NE) et un oligonucléotide simple brin (40) complémentaire à la séquence d'étiquette universelle (16), dans lequel la séquence d'étiquette universelle (16) comprend un site de reconnaissance pour ladite endonucléase de coupure (NE).

11. Kit pour détecter l'amplification d'une séquence d'acide nucléique cible (10) dans un échantillon résultant d'une réaction d'amplification d'acide nucléique capable de générer un produit d'amplification (18b, 18c) comprenant à une extrémité une séquence d'étiquette universelle (16) ayant une longueur comprise entre 8 et 30 nucléotides, le kit étant **caractérisé en ce qu'**il comprend :
- des nanoparticules d'or colloïdal (AuNP1, AuNP3, AuNP5, AuNP7) fonctionnalisées avec une première sonde oligonucléotidique (20, 24, 30, 34) ;
- des nanoparticules d'or colloïdal (AuNP2, AuNP4, AuNP6, AuNP8) fonctionnalisées avec une seconde sonde oligonucléotidique (22, 26, 32, 36) ;
dans lequel ladite première sonde oligonucléotidique (20, 24, 30, 34) est au moins partiellement complémentaire à une première partie de ladite séquence d'étiquette universelle (16) et ladite seconde sonde oligonucléotidique (22, 26, 32, 36) est au moins partiellement complémentaire à une seconde partie de ladite séquence d'étiquette universelle (16) ou ladite seconde sonde oligonucléotidique (22, 26, 32, 36) est au moins partiellement complémentaire à ladite première sonde oligonucléotidique (20, 24, 30, 34),
**caractérisé en ce qu'**il comprend également une première enzyme de restriction (R1) conçue pour cliver la séquence d'étiquette universelle (16) au niveau d'un un premier site de restriction, et facultativement une seconde enzyme de restriction (R2) conçue pour cliver la séquence d'étiquette universelle (16) au niveau d'un second site de restriction, la séquence d'étiquette universelle (16) comprenant ledit premier site de restriction et facultativement ledit second site de restriction.

12. Kit selon les revendications 10 ou 11, dans lequel la longueur de chacune desdites première et seconde sondes oligonucléotidiques (20, 24, 30, 34 ; 22, 26, 32, 36) avec lesquelles les particules d'or colloïdal sont fonctionnalisées est comprise entre 5 et 80 nucléotides, de préférence entre 15 et 40 nucléotides.

13. Kit selon l'une quelconque des revendications 10 à 12, dans lequel lesdites nanoparticules d'or colloïdal présentent une taille particulaire comprise entre 1 et 500 nm, de préférence entre 15 et 80 nm.

14. Kit selon l'une quelconque des revendications 10 à 13, dans lequel lesdites nanoparticules d'or colloïdal fonctionnalisées avec une première sonde oligonucléotidique et lesdites nanoparticules d'or colloïdal fonctionnalisées avec une seconde sonde oligonucléotidique présentent une densité de fonctionnalisation de 2x10⁻⁴ à 2x10⁻¹/nm², de préférence de 1x10⁻³/nm² à 8x10⁻²/nm².
